# FASCICULE DE BREVET EUROPEEN

(11) **EP 1 468 322 B1**
(45) Date de publication et mention de la délivrance du brevet: **03.08.2005**
(21) Numéro de dépôt: 02805803.0
(22) Date de dépôt: 20.12.2002
(51) Int. Cl.: G02B 23/24, A61B 1/00

(54) **APPAREILLAGE D IMAGERIE CONFOCALE NOTAMMENT POUR ENDOSCOPE**
KONFOKALE ABBILDUNGSGERÄTE INSBESONDERE FÜR EIN ENDOSKOP
CONFOCAL IMAGING EQUIPMENT IN PARTICULAR FOR ENDOSCOPE

(30) Priorité: 28.12.2001 FR 0116980
(43) Date de publication de la demande: 20.10.2004
(73) Titulaire: Mauna Kea Technologies, 75010 Paris (FR)
(72) Inventeur: VIELLEROBE, Bertrand, F-94130 Nogent sur Marne (FR); GENET, Magalie, F-78280 Guyancourt (FR); BERIER, Frédéric, F-92400 Courbevoie (FR); LACOMBE, François, F-92370 Chaville (FR); PERCHANT, Aymeric, F-75013 Paris (FR); LE GOUALHER, Georges, F-35520 La Meziere (FR); MARTI, Sandra, F-92000 Nanterre (FR); BOURRIAUX, Stéphane, F-77420 Champs sur Marne (FR)
(74) Mandataire: Pontet, Bernard
(86) Numéro de dépôt international: PCT/FR2002/004481
(87) Numéro de publication internationale: WO 2003/056378

(56) Documents cités:
- WO-A-00/16151
- WO-A-01/44854
- US-A- 5 995 867
- US-A1- 2001 043 383
- US-B1- 6 208 886

## Description

La présente invention concerne un appareillage d'imagerie confocale notamment pour endoscope et du type utilisant un faisceau de fibres optiques souples. Le caractère confocal réside dans l'utilisation du même chemin à l'illumination et à la détection, et dans la filtration spatiale du signal revenant du plan d'analyse subsurfacique.

Les domaines d'applications de l'invention sont l'analyse de tissus biologiques in-vivo sur l'homme ou l'animal, externes par exemple dans le domaine de la dermatologie, ou internes et accessibles à l'aide d'un canal opérateur d'endoscope dans lequel on peut introduire le faisceau de fibres optiques souples, et également l'analyse ex-vivo d'échantillons tissulaires provenant de prélèvements biopsiques, et l'analyse in-vitro de culture en biologie cellulaire. En outre encore, le dispositif peut servir à l'analyse de l'intérieur d'un dispositif manufacturé.

Actuellement sont visés les domaines médicaux de la gastroentérologie, la pneumologie, la gynécologie, l'urologie, l'ORL, la dermatologie, l'ophtalmologie, la cardiologie et de la neurologie.

La mise en oeuvre d'un faisceau de fibres optiques souples de petit diamètre (plusieurs centaines de microns) est nécessaire pour un couplage avec le canal opérateur d'un endoscope mais elle peut être également avantageuse pour des systèmes de tests automatiques dans lesquels le faisceau de fibres optiques, avec à son extrémité une tête optique de focalisation, est manipulé de manière automatisée comme un bras de mesure sur une matrice d'échantillon. Par ailleurs, indépendamment d'une application endoscopique, une miniaturisation de la tête optique est également avantageuse pour augmenter la précision du positionnement et également pour minimiser l'inertie mécanique dans les applications automatisées.

Plus particulièrement, l'appareillage selon l'invention est du type comprenant une source émettant une radiation à une longueur d'onde donnée produisant un faisceau d'illumination parallèle. Ce faisceau d'illumination est ensuite séparé par exemple par une lame séparatrice afin de dissocier la voie d'illumination et la voie de détection. II est ensuite dévié angulairement dans deux directions de l'espace (balayage) par un système optomécanique de miroirs. Un moyen optique reprend ensuite le faisceau balayé angulairement et l'injecte dans un guide d'image situé dans le plan focal de ce dernier et constitué d'un faisceau ordonné de plusieurs dizaines de milliers de fibres optiques souples. On injecte ainsi, à un instant donné, une des fibres optiques du guide d'image pour une position angulaire donnée du faisceau. Au cours du temps, on injecte successivement les fibres optiques constituant le guide d'image en déviant angulairement le faisceau au moyen des miroirs, et ce point par point pour une ligne donnée, et ligne après ligne pour constituer l'image. Le faisceau injecté dans le guide d'image (le cas échéant préalablement disposé dans le canal opérateur d'un endoscope) est guidé, en émerge et est repris par un moyen optique permettant d'illuminer point à point le site que l'on souhaite observer. A chaque instant, le spot illuminant le tissu est rétrodiffusé et suit le trajet inverse du faisceau incident. Ce flux rétrodiffusé est donc réinjecté dans le guide d'image, en émerge, atteint le système de balayage, est ensuite renvoyé sur la voie de détection au moyen de la lame séparatrice, puis focalisé dans un trou de filtrage. Il est alors détecté par exemple par un photomultiplicateur ou une photodiode à avalanche. Le signal issu du photodétecteur est ensuite intégré, puis numérisé pour être visualisé sur un écran.

Un dispositif de ce type est décrit notamment dans la demande de brevet internationale WO 00/16151.

Dans le cas de l'analyse d'un tissu biologique, les difficultés que l'on rencontre sont liées au faible rapport du signal utile rétrodiffusé sur le signal parasite, qui nécessite, pour que l'image produite soit acceptable, une qualité de faisceau d'illumination la meilleure possible et conservée tout au long du trajet optique, notamment au niveau de la qualité du front d'onde et de la répartition spatiale de l'intensité de la tache focale qui doit être la plus proche possible du diamètre de coeur d'une fibre. Du côté de l'extrémité proximale du guide d'image, la dégradation du faisceau d'illumination tant sur le plan énergétique que spatial est notamment due aux réflexions parasites qui s'opèrent à l'entrée du guide d'image et aux défauts de transmission optique au niveau des systèmes de balayage et d'injection (déformation de champ, erreur du front d'onde).

Dans la demande de brevet internationale WO 00/16151 mentionnée ci-dessus, le système de balayage comprend des miroirs résonnants optomécaniques et/ou galvanométriques et le système d'injection dans le guide d'image une lentille L4 de focalisation ou un objectif de microscope.

Le document US 5 995 867 décrit un système pour réaliser de la chirurgie cellulaire comprenant un faisceau laser et des moyens d'optique confocale pour balayer et focaliser le faisceau dans le tissu et générer en retour des images confocales. Ce système ne comprend pas de guide d'image constitué de fibres optiques souples et a fortiori ne décrit pas de moyens d'injection du faisceau balayé tour à tour dans une fibre.

La présente invention a pour but de proposer un appareillage dont la qualité du faisceau d'illumination est améliorée à l'entrée du guide d'image et par conséquent la qualité d'image est améliorée aussi. Elle a également pour but de proposer une solution pour cela à faible coût, simple à mettre en oeuvre, miniaturisable et industrialisable.

Elle propose un appareillage d'imagerie confocale selon la revendication 1.

Grâce à ces moyens optiques, on peut garantir une qualité du faisceau d'illumination et un taux de couplage fibre à fibre homogène et optimal.

Selon un exemple particulier un système optique d'afocaux comprend quatre lentilles dont un doublet correcteur placé symétriquement par rapport au plan image permettant de corriger la courbure de champ et de minimiser l'erreur du front d'onde.

Pour minimiser encore les aberrations résiduelles, les moyens d'injection dans le guide d'image comprennent un jeu de lentilles pour transformer le balayage angulaire du faisceau d'illumination en un balayage en translation du guide d'image qui comporte en amont un doublet adapté à corriger la courbure de champ résiduelle dudit jeu de lentilles.

De manière avantageuse selon l'invention, les moyens électroniques de commande, d'analyse et de traitement numérique du signal détecté et de visualisation comprennent une carte de synchronisation adaptée notamment à commander de manière synchronisée le mouvement des miroirs ligne et trame et adaptée à connaître à tout instant la position du faisceau d'illumination balayé.

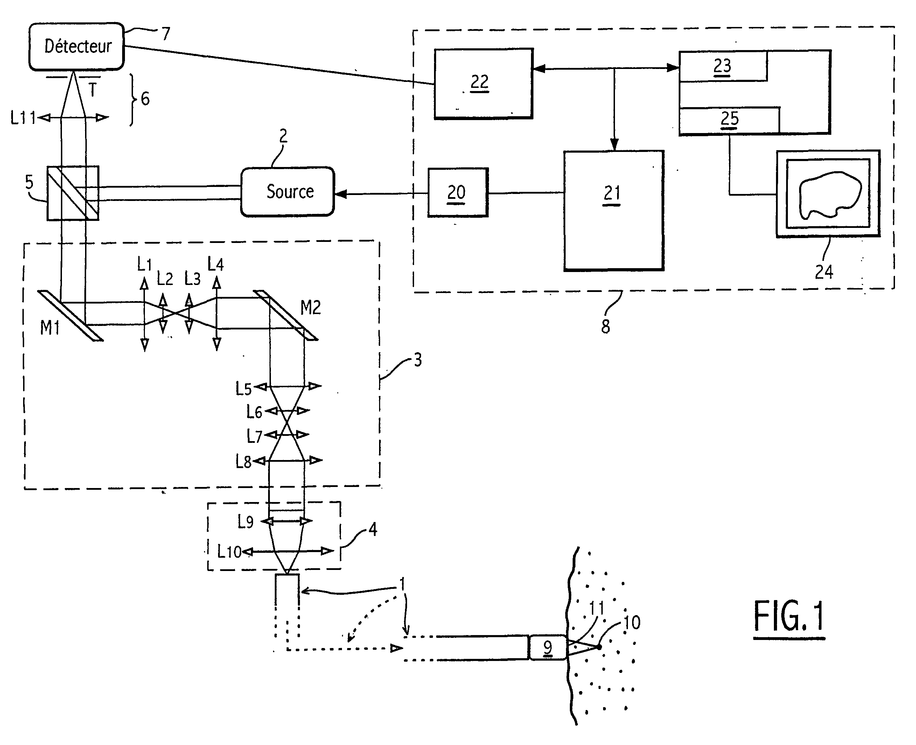

La présente invention sera mieux comprise et d'autres avantages apparaîtront à la lumière de la description qui va suivre d'un exemple de réalisation, description faite en référence à la figure 1 sur laquelle est représenté schématiquement un appareillage selon ledit exemple.

Sur la figure 1, il est proposé un appareillage pour réaliser une image d'un site situé à une profondeur donnée dans un plan P de coupe XY perpendiculaire à l'axe optique, ledit appareillage comportant un guide d'image 1 constitué de plusieurs dizaines de milliers de fibres optiques souples avec :
- du côté de l'extrémité proximale du guide d'image 1 : une source 2 produisant un faisceau d'illumination, des moyens de balayage angulaire 3 dudit faisceau, des moyens d'injection 4 du faisceau dévié tour à tour dans l'une des fibres du guide d'image 1, des moyens de séparation 5 du faisceau d'illumination et du signal rétroémis, des moyens de filtrage spatial 6, des moyens de détection 7 dudit signal, des moyens électroniques 8 de commande, d'analyse et de traitement numérique du signal détecté et de visualisation ; et
- du côté de l'extrémité distale du guide d'image 1 : une tête optique 9 adaptée à focaliser le faisceau d'illumination sortant de la fibre illuminée du guide d'image en un point focalisé 10 dans le plan P sous la zone de contact 11 de la tête optique 9.

Tous ces moyens sont décrits ci-après en détails.

Le guide d'image 1 permet d'accéder à la zone d'analyse subsurfacique en déportant la source 2. S'il est destiné, avec la tête optique 9, à être inséré dans le canal opérateur de l'endoscope, il doit présenter des dimensions qui soient compatibles (quelques millimètres de diamètre suivant l'application clinique). Il est constitué d'un faisceau ordonné de fibres optiques souples entouré d'une gaine. On peut utiliser tout guide présentant suffisamment de fibres et un faible espacement inter-coeur afin d'obtenir une bonne résolution spatiale. A titre d'exemple, on peut utiliser un guide de marque Sumitomo® constitué de 30 000 fibres de diamètre de coeur de 2,5 µm et d'espacement inter-coeur de 4 µm, ou bien un guide de marque Fujikura® constitué de 30 000 fibres de diamètre de coeur de 2µm et d'espacement inter-coeur de 3,7 µm. Selon l'invention, les fibres sont illuminées une à une tour à tour et de manière adressée, grâce aux moyens de balayage 3 et aux moyens d'injection 4. Le diamètre utile du guide d'image correspond donc au diamètre de coeur d'une fibre illuminée.

Le guide d'image 1 est équipé à ses deux extrémités d'une lame de verre (non représentées sur la figure) suffisamment épaisse afin de rejeter les réflexions parasites en dehors des moyens de filtrage 6 pour la réflexion qui s'opère à l'entrée du faisceau de fibres, et en dehors de la fibre optique illuminée pour la réflexion qui s'opère en sortie du guide d'image. Les lames de verre sont traitées anti-reflet afin de minimiser la lumière réfléchie.

La source 2 est constituée d'une diode laser à 683 nm devant présenter une très bonne qualité de front d'onde, inférieure ou égale à λ/10. Selon l'invention, cette diode est pulsée afin de dissocier par détection synchrone le signal utile de la réflexion parasite qui s'opère à l'entrée du guide d'image 1. En variante, on peut utiliser un laser solide ou à gaz, mais le choix en longueur d'onde dans la bande 600-800 nm où l'absorption dans les tissus est moindre, est moins étendu; de plus, le coût à puissance équivalente est bien plus important.

Les moyens 5 pour séparer le faisceau d'illumination et le signal retour sont constitués ici d'un cube séparateur 50/50 pour des commodités de réglage. On peut aussi utiliser une lame séparatrice 50/50.

Les moyens de balayage 3 ont pour fonction de reproduire une matrice de diodes de même qualité optique que la diode laser de la source 2 et que l'on injectera fibre à fibre. Ceci nécessite une combinaison de moyens optiques non standards permettant de corriger les aberrations présentes dans le système de transport et de duplication de source afin d'éclairer le guide de signal fibre par fibre. Le système de balayage est constitué de deux miroirs M1 et M2 et de deux systèmes optiques. Le miroir M1 est un miroir « ligne » résonant à une fréquence de 4 kHz et le miroir M2 un miroir « trame », galvanométrique avec une fréquence variable entre 0 et 300 Hz. Chaque système optique est constitué de quatre lentilles, respectivement, L1-L4 et L5-L8, permettant de conjuguer les deux miroirs dans un premier temps, puis le miroir M2 et l'entrée du guide d'image. Ces systèmes optiques ne doivent pas présenter d'aberrations qui pourraient :
- élargir la répartition spatiale de l'intensité de la tache focale (FEP : Fonction d'Etalement du Point ou PSF : « Point Spread Function » en langue anglaise) après les moyens d'injection 4 et ainsi dégrader le couplage dans le guide d'image 1;
- faire propager le flux dans la gaine du guide d'image 1 qui dégraderait la PSF en bout de guide et de ce fait la résolution de l'image.
Les lentilles L2-L3 et L6-L7 sont des doublets correcteurs identiques placés symétriquement par rapport au plan image. Cela permet d'homogénéiser l'injection dans le guide d'image en corrigeant la courbure de champ et en minimisant l'erreur du front d'onde dues à l'utilisation de systèmes afocaux hors d'axes (L1-L4 et L5-L8).

Les moyens d'injection 4 : Ils doivent présenter le minimum d'aberrations et ne doivent pas dégrader la qualité du front d'onde afin de réaliser une tache de focalisation proche de la limite de diffraction pour ainsi réaliser un couplage optimal avec la fibre adressée (une PSF égale au diamètre de coeur d'une fibre). Ils comprennent un doublet sur-mesure L9 et un triplet standard L10. Le doublet L9 permet de corriger les aberrations résiduelles du triplet L10, à savoir la courbure de champ.

Les moyens de filtrage spatial 6 comprennent une lentille L11 et un trou de filtrage T permettant de ne sélectionner que la fibre d'illumination et non les fibres adjacentes qui peuvent générer un signal parasite. La taille du trou de filtrage est telle qu'elle correspond au diamètre de coeur d'une fibre au grandissement près du système optique entre l'entrée du faisceau de fibres et le trou de filtrage.

La tête optique 9 comprend plusieurs moyens optiques permettant de faire converger le faisceau émergeant de la fibre optique illuminée et deux lames de verre, l'une est celle décrite plus haut en sortie du guide d'image et l'autre est un hublot adapté à venir en contact du site et réalisant une adaptation d'indice. Les moyens optiques présentent les caractéristiques suivantes :
- permettre une analyse du tissu à une profondeur de plusieurs dizaines à plusieurs centaines de microns ;
- minimiser les aberrations afin de transcrire la PSF en sortie du guide d'image sur le tissu sans élargir celle-ci ou la déformer ;
- optimiser le taux de couplage en retour dans le guide d'image en optimisant la qualité du front d'onde ;
- le cas échant, des dimensions compatibles avec celles du canal opérateur d'un endoscope.

Les moyens optiques comprennent par exemple un système de lentilles formant un objectif sur-mesure.

Les moyens de détection 7 comprennent comme détecteur de signal une photodiode à avalanche qui acquiert le signal en continu, le signal parasite provenant des deux extrémités du guide de signal étant ramené au même ordre de grandeur que le signal utile afin de ne pas saturer le détecteur. La suppression du résidu de réflexion parasite à l'entrée du guide d'image est ensuite effectuée par un filtrage temporel numérique.

Les moyens électroniques 8 de commande, d'analyse et de traitement numérique du signal détecté et de visualisation comprennent les cartes suivantes:
- une carte de modulation 20 de la source laser. Cette carte permet de moduler la source à une fréquence relativement élevée (de l'ordre de 100 MHz) afin de produire des impulsions (10 ns ≤ τ ≤ 100 ns) à intervalles réguliers (rapport cyclique de l'ordre de 4).
- une carte de synchronisation 21 qui a pour fonctions :
   - de commander de manière synchronisée le balayage, c'est-à-dire le mouvement des miroirs ligne M1 et trame M2 ;
   - de connaître à tout instant la position du spot laser ainsi balayé ;
   - de synchroniser l'émission des impulsions de la source laser avant la détection ;
   - de gérer toutes les autres cartes par l'intermédiaire d'un micro-contrôleur lui-même pouvant être piloté ;
- une carte détecteur 22 qui comprend un circuit analogique qui réalise notamment une adaptation d'impédance et une intégration, un convertisseur analogique numérique puis un composant logique programmable (par exemple un circuit FGPA) qui met en forme le signal ;
- une carte d'acquisition numérique 23 qui permet de traiter un flot de données numériques à fréquence variable et de l'afficher sur un écran 24 ;
- une carte graphique 25.
Le traitement d'image se fait de la manière suivante. L'information brute en sortie de carte de détecteur est mise en forme et traitée afin d'être visualisable puis interprétable. Le procédé d'acquisition des images à travers le guide d'image constitué de plusieurs dizaines de milliers de fibres optiques et par balayage de ce dernier induit des spécificités dans l'image et un traitement approprié.

Deux groupes de traitements sont prévus :
1. Le premier groupe est constitué de procédés de traitement de signal visant à calibrer le signal acquis. On peut ainsi s'affranchir des défauts de couplage laser/guide inhérent au procédé d'acquisition, ainsi que des défauts dus à certains bruits du système. La calibration peut prendre différentes formes suivant la précision du contrôle de balayage, et sa stabilité dans le temps. Ces traitements sont essentiellement monodimensionnels.
2. Le second groupe permet d'améliorer l'interprétation en intégrant des traitements d'image (2D et 2D+temps) spécifiques au procédé optomécanique. Ces traitements consistent en un procédé de restauration d'images, suivis d'un procédé de recalage rapide permettant de s'affranchir des petits mouvements. Ces traitements sont rapides par rapport à la durée d'acquisition. Ces algorithmes sont entièrement automatiques et s'adaptent à la nature de l'image.

Il va de soi que des variantes de réalisation sont possibles notamment en ce qui concerne le miroir ligne M1 qui peut résonner à une autre fréquence par exemple 8kHz, les systèmes optiques d'afocaux qui peuvent être entièrement sur-mesure ou bien comporter d'autres jeux de lentilles correctrices adaptées.

## Revendications

1. Appareillage d'imagerie confocale notamment pour endoscope comportant un guide d'image (1) constitué de fibres optiques souples avec :
- du côté de l'extrémité proximale du guide d'image (1) : une source (2) produisant un faisceau d'illumination, des moyens de balayage angulaire (3) dudit faisceau, des moyens d'injection (4) du faisceau dévié tour à tour dans l'une des fibres du guide d'image (1), des moyens de séparation (5) du faisceau d'illumination et du signal rétroémis, des moyens de filtrage spatial (6), des moyens de détection (7) dudit signal, des moyens électroniques (8) de commande, d'analyse et de traitement numérique du signal détecté et de visualisation ; et
- du côté de l'extrémité distale du guide d'image (1) : une tête optique (9) adaptée à focaliser le faisceau d'illumination sortant de la fibre illuminée, **caractérisé en ce que** les moyens de balayage angulaire (3) comprennent un miroir ligne résonnant (M1) et un miroir trame (M2) galvanométrique avec une fréquence variable et deux systèmes optiques d'afocaux adaptés à conjuguer les deux miroirs (M1,M2) dans un premier temps puis le miroir trame (M2) et le moyen d'injection (4) dans le guide d'image dans un deuxième temps, chaque système optique respectant la qualité du front d'onde (WFE) initiale et présentant une répartition spatiale de l'intensité de la tache focale (PSF) égale au diamètre de coeur d'une fibre; et **en ce qu'**un système optique d'afocaux comprend des lentilles standards et des lentilles correctrices adaptées à corriger les aberrations résiduelles desdites lentilles standards.

2. Appareillage selon la revendication 1, **caractérisé en ce que** le système optique d'afocaux comprend quatre lentilles (L1-L4 ; L5-L8) dont un doublet correcteur (L2,L3 ; L6,L7) placé symétriquement par rapport au plan image permettant de corriger la courbure de champ et de minimiser l'erreur du front d'onde.

3. Appareillage selon la revendication 1 ou 2, **caractérisé en ce que** les moyens d'injection (4) comprennent un jeu de lentilles (L10) adapté à transformer le balayage angulaire en un balayage en translation du guide d'image et en amont un doublet (L9) adapté à corriger la courbure de champ résiduelle dudit jeu de lentilles (L10).

4. Appareillage selon la revendication 3, **caractérisé en ce que** ledit jeu de lentilles (L10) est un triplet.

5. Appareillage selon l'une des revendications précédentes, **caractérisé en ce qu'**il comprend une lame de verre ménagée à l'entrée du guide d'image destinée à rejeter les réflexions parasites en dehors des moyens de filtrage (6).

6. Appareillage selon l'une des revendications précédentes, **caractérisé en ce qu'**il comprend une lame de verre ménagée à la sortie du guide d'image destinée à rejeter les réflexions parasites en dehors de la fibre optique illuminée.

7. Appareillage selon l'une des revendications précédentes, **caractérisé en ce que** le miroir ligne (M1) est un miroir résonnant à une fréquence de 4 kHz.

8. Appareillage selon l'une quelconque des revendications précédentes, **caractérisé en ce que** le miroir trame (M2) a une fréquence variable entre 0 et 300 Hz.

9. Appareillage selon l'une quelconque des revendications précédentes, **caractérisé en ce que** les moyens électroniques (8) de commande, d'analyse et de traitement numérique du signal détecté et de visualisation comprennent une carte de synchronisation (21) adaptée notamment à commander de manière synchronisée le mouvement des miroirs ligne (M1) et trame (M2) et adaptée à connaître à tout instant la position du faisceau d'illumination balayé.

## Patentansprüche

1. Konfokales Abbildungsgerät insbesondere für Endoskop, umfassend einen aus biegsamen optischen Fasern bestehenden Bildleiter (1) mit:
- auf der Seite des proximalen Endes des Bildleiters (1): einer einen Beleuchtungsstrahl erzeugenden Quelle (2), Mitteln (3) zur Winkelablenkung des Strahls, Mitteln (4) zur Einspeisung des nacheinander abgelenkten Strahls in eine der Fasern des Bildleiters (1), Mitteln (5) zur Trennung des Beleuchtungsstrahls und des zurückgesendeten Signals, Mitteln (6) zur räumlichen Filterung, Mitteln (7) zur Erfassung des Signals, elektronischen Mitteln (8) zur Steuerung, zur Analyse und zur digitalen Verarbeitung des erfassten Signals und zur Sichtdarstellung; und
- auf der Seite des distalen Endes des Bildleiters (1): einem Optikkopf (9), der dafür ausgelegt ist, den aus der beleuchteten Faser austretenden Beleuchtungsstrahl zu fokussieren,
**dadurch gekennzeichnet, dass** die Winkelablenkungsmittel (3) einen Zeilen-Resonanzspiegel (M1) und einen galvanometrischen Raster-Spiegel (M2) mit einer veränderlichen Frequenz und zwei optische Afokalsysteme umfassen, die dafür ausgelegt sind, in einem ersten Takt die beiden Spiegel (M1, M2) und dann in einem zweiten Takt den Raster-Spiegel (M2) und das Mittel (4) zur Einspeisung in den Bildleiter zu koppeln, wobei jedes optische System die ursprüngliche Qualität der Wellenfront (WFE) wahrt und eine räumliche Verteilung der Intensität des Brennflecks (PSF) gleich dem Kerndurchmesser einer Faser aufweist, und dass ein optisches Afokalsystem Standardlinsen und Korrekturlinsen umfasst, die dafür ausgelegt sind, die Restaberrationen der Standardlinsen zu korrigieren.

2. Gerät nach Anspruch 1, **dadurch gekennzeichnet, dass** das optische Afokalsystem vier Linsen (L1-L4; L5-L8) umfasst, darunter ein bezüglich der Bildebene symmetrisch angeordnetes Korrekturdublett (L2, L3; L6, L7), das die Korrektur der Feldkrümmung und die Minimierung der Abweichung der Wellenfront gestattet.

3. Gerät nach Anspruch 1 oder 2, **dadurch gekennzeichnet, dass** die Einspeisungsmittel (4) einen Linsensatz (L10), der dafür ausgelegt ist, die Winkelablenkung in eine Translationsablenkung des Bildleiters umzuwandeln, und stromaufwärts ein Dublett (L9) umfassen, das dafür ausgelegt ist, die Restfeldkrümmung dieses Linsensatzes (L10) zu korrigieren.

4. Gerät nach Anspruch 3, **dadurch gekennzeichnet, dass** der Linsensatz (L10) ein Triplett ist.

5. Gerät nach einem der vorhergehenden Ansprüche, **dadurch gekennzeichnet, dass** es eine am Eintritt des Bildleiters angeordnete Glasplatte aufweist, die dazu bestimmt ist, die Störreflexionen aus den Filtermitteln (6) heraus auszuscheiden.

6. Gerät nach einem der vorhergehenden Ansprüche, **dadurch gekennzeichnet, dass** es eine am Austritt des Bildleiters angeordnete Glasplatte aufweist, die dazu bestimmt ist, die Störreflexionen aus der beleuchteten optischen Faser heraus auszuscheiden.

7. Gerät nach einem der vorhergehenden Ansprüche, **dadurch gekennzeichnet, dass** der Zeilen-Spiegel (M1) ein mit einer Frequenz von 4 kHz resonierender Spiegel ist.

8. Gerät nach einem der vorhergehenden Ansprüche, **dadurch gekennzeichnet, dass** der Raster-Spiegel (M2) eine zwischen 0 und 300 Hz veränderliche Frequenz hat.

9. Gerät nach einem der vorhergehenden Ansprüche, **dadurch gekennzeichnet, dass** die elektronischen Mittel (8) zur Steuerung, zur Analyse und zur digitalen Verarbeitung des erfassten Signals und zur Sichtdarstellung eine Synchronisationskarte (21) umfassen, die insbesondere dafür ausgelegt ist, die Bewegung des Zeilen-Spiegels (M1) und des Raster-Spiegels (M2) synchron zu steuern, und dafür ausgelegt ist, zu jedem Zeitpunkt die Kenntnis der Stellung des abgelenkten Beleuchtungsstrahls zu gestatten.

## Claims

1. Confocal imaging equipment in particular for endoscope comprising an image guide (1) constituted by flexible optical fibres with:
- on the side of the proximal end of the image guide (1): a source (2) producing an illumination beam, means for angular scanning (3) of said beam, means for injecting (4) the beam deflected alternately into one of the fibres of the image guide (1), means for separating (5) the illumination beam and the backscattered signal, means for spatial filtering (6), means for detecting (7) said signal, electronic means (8) for controlling, analyzing and digital processing of the detected signal and display; and
- on the side of the distal end of the image guide (1): an optical head (9) adapted for focussing the illumination beam leaving the illuminated fibre, **characterized in that** the means for angular scanning (3) comprise a resonating line mirror (M1) and a galvanometric frame mirror (M2) with a variable frequency and two afocal optical systems adapted for conjugating first the two mirrors (M1, M2) then for conjugating the frame mirror (M2) and the injection means (4) in the image guide, each optical system respecting the initial quality of the wave front (WFE) and having a spatial distribution of the focal spot intensity (PSF) equal to the diameter of a fibre core; and **in that** an afocal optical system comprises standard lenses and corrective lenses adapted for correcting the residual aberrations of said standard lenses.

2. Equipment according to claim 1, **characterized in that** the afocal optical system comprises four lenses (L1-L4; L5-L8) a corrective doublet (L2, L3; L6, L7) of which is placed symmetrically relative to the image plane allowing correction of the curvature of field and minimization of the wave front error.

3. Equipment according to claim 1 or 2, **characterized in that** the injection means (4) comprise a set of lenses (L10) adapted for converting
the angular scanning to translational scanning of the image guide and upstream a doublet (L9) adapted for correcting the residual curvature of field of said set of lenses (L10).

4. Equipment according to claim 3, **characterized in that** said set of lenses (L10) is a triplet.

5. Equipment according to one of the preceding claims, **characterized in that** it comprises a glass plate arranged at the image guide input intended to reject the parasitic reflections outside the filtering means (6).

6. Equipment according to one of the preceding claims, **characterized in that** it comprises a glass plate arranged at the image guide output intended to reject the parasitic reflections outside the illuminated optical fibre.

7. Equipment according to one of the preceding claims, **characterized in that** the line mirror (M1) is a mirror resonating at a frequency of 4 kHz.

8. Equipment according to any one of the preceding claims, **characterized in that** the frame mirror (M2) has a variable frequency between 0 and 300 Hz.

9. Equipment according to any one of the preceding claims, **characterized in that** the electronic means (8) for controlling, analyzing and digital processing of the detected signal and display comprise a synchronization card (21) adapted in particular for controlling in a synchronized manner the movement of the line mirror (M1) and frame mirror (M2) and adapted to know at any moment the position of the scanned illumination beam.
